(19) **Europäisches Patentamt / European Patent Office / Office européen des brevets**

(11) **EP 4 463 693 B1**

(12) **FASCICULE DE BREVET EUROPEEN**

(45) Date de publication et mention de la délivrance du brevet:
**16.07.2025 Bulletin 2025/29**

(21) Numéro de dépôt: **23700734.9**

(22) Date de dépôt: **11.01.2023**

(51) Classification Internationale des Brevets (IPC):
**G01N 21/552** (2014.01)

(52) Classification Coopérative des Brevets (CPC):
**G01N 21/553**

(86) Numéro de dépôt international:
**PCT/EP2023/050532**

(87) Numéro de publication internationale:
**WO 2023/135163 (20.07.2023 Gazette 2023/29)**

(54) **PROCEDE DE DETECTION D'OBJETS BIOLOGIQUES PAR IMAGERIE PAR RESONANCE PLASMONIQUE DE SURFACE**

VERFAHREN ZUR DETEKTION BIOLOGISCHER OBJEKTE MITTELS OBERFLÄCHENPLASMONENRESONANZBILDGEBUNG

METHOD FOR DETECTING BIOLOGICAL OBJECTS BY MEANS OF SURFACE PLASMON RESONANCE IMAGING

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **14.01.2022 FR 2200324**

(43) Date de publication de la demande:
**20.11.2024 Bulletin 2024/47**

(73) Titulaires:
- **Aryballe**
  **38000 Grenoble (FR)**
- **Commissariat à l'Energie Atomique et aux Energies Alternatives**
  **75015 Paris (FR)**
- **Centre National de la Recherche Scientifique**
  **75016 Paris 16 (FR)**
- **Université Grenoble Alpes**
  **38400 Saint Martin d'Hères (FR)**

(72) Inventeurs:
- **ANDREI, Cristina-Cassiana**
  **38000 GRENOBLE (FR)**
- **HERRIER, Cyril**
  **38000 GRENOBLE (FR)**
- **SLIMANI, Sami**
  **38000 GRENOBLE (FR)**
- **HOU-BROUTIN, Yanxia**
  **38044 GRENOBLE (FR)**
- **MOREAU, Christophe**
  **38044 GRENOBLE CEDEX 9 (FR)**
- **LIVACHE, Thierry**
  **38000 GRENOBLE (FR)**

(74) Mandataire: **Ipside**
  **6, Impasse Michel Labrousse**
  **31100 Toulouse (FR)**

(56) Documents cités:
**FR-A1- 2 959 568      FR-A1- 3 103 895**

- **SOPHIE BRENET ET AL: "Highly-Selective Optoelectronic Nose Based on Surface Plasmon Resonance Imaging for Sensing Volatile Organic Compounds", ANALYTICAL CHEMISTRY, vol. 90, no. 16, 19 July 2018 (2018-07-19), US, pages 9879 - 9887, XP055630144, ISSN: 0003-2700, DOI: 10.1021/acs.analchem.8b02036**

**Description**

**DOMAINE TECHNIQUE**

Le domaine de l'invention est celui de la détection d'objets biologiques au moyen d'un système de détection par imagerie par résonance plasmonique de surface. Les objets biologiques peuvent être de petites tailles, par exemple de l'ordre d'une centaine de nanomètres à quelques micromètres, notamment au regard de la résolution spatiale du système de détection.

**ÉTAT DE LA TECHNIQUE ANTÉRIEURE**

La capacité de détecter des objets biologiques notamment de petites tailles, par exemple des microorganismes tels que des virus ou des bactéries, est une problématique de plus en plus importante, notamment dans le domaine de la santé, comme dans celui de l'agroalimentaire ou de l'environnement. La détection de tels objets biologiques peut être effectuée par un système de détection par imagerie de résonance plasmonique de surface (SPRi pour *Surface Plasmon Resonance Imaging,* en anglais), qui présente l'avantage d'être une technique de détection dite sans marqueur (*label free,* en anglais) où les objets biologiques ne sont pas « marqués » au préalable par un agent de révélation.

La résonance plasmonique de surface se produit lorsqu'un signal lumineux éclaire une interface métal-diélectrique sous certaines conditions de longueur d'onde, de polarisation et d'angle d'incidence. Cette interface peut être formée par une fine couche métallique située sur la surface d'un prisme, et par le fluide contenant les analytes. Elle peut comporter des ligands adaptés à se lier de manière spécifique aux analytes, et forme ainsi une surface fonctionnalisée. Lorsque les conditions sont respectées, les électrons libres à la surface de la couche métallique absorbent les photons incidents et les convertissent en ondes de plasmons de surface. Ces conditions de résonance plasmonique dépendent notamment de l'indice de réfraction à la surface de la couche métallique. Ainsi, lorsqu'une interaction d'adsorption/désorption se produit entre un ligand et un analyte, l'indice de réfraction change et les conditions de résonance plasmonique sont modifiées. Il est alors possible de suivre en temps réel les interactions d'adsorption/désorption sans aucun marquage.

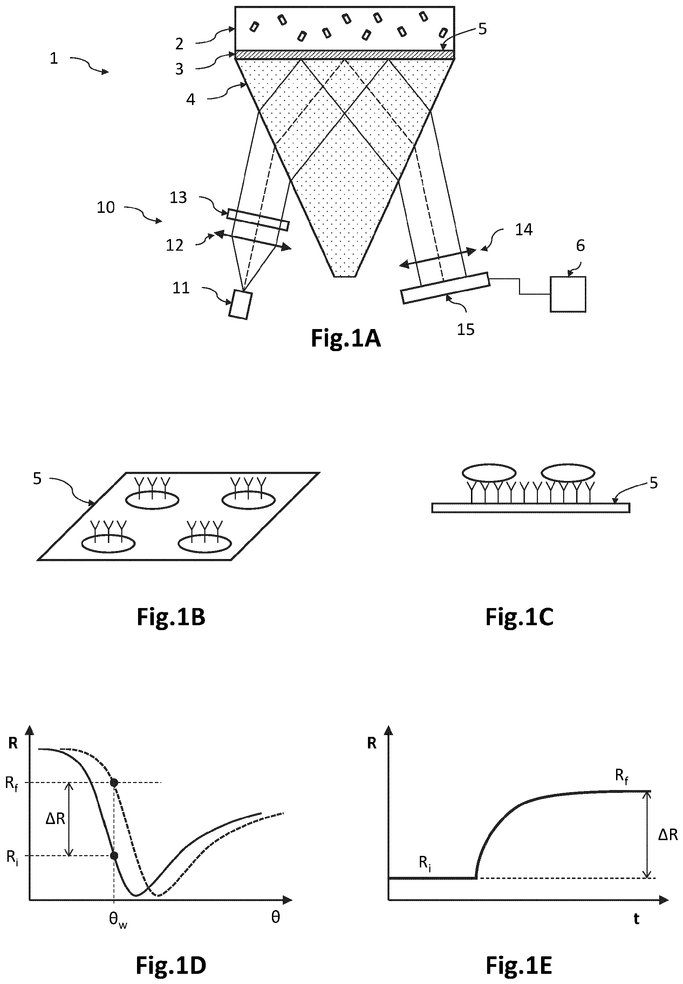

La figure 1A est une vue schématique et partielle d'un système de détection 1 par imagerie SPR selon un exemple de l'art antérieur décrit notamment dans le document WO2018/158458A1. Il comporte une surface fonctionnalisée 5, située sur une face d'un prisme 4, comportant une pluralité de sites sensibles adaptés à capter par adsorption des objets biologiques présents dans un échantillon liquide, et un dispositif optique de mesure 10 adapté à acquérir une image des sites sensibles. Il comporte en outre une unité de traitement 6 permettant par exemple de détecter la présence d'objets biologiques à partir des images fournies par le dispositif optique de mesure 10. Un dispositif de gestion fluidique (non représenté) peut être prévu pour amener l'échantillon liquide au contact de la surface fonctionnalisée 5. Le dispositif optique de mesure 10 comporte une source optique 11, un dispositif optique de mise en forme formé ici d'une lentille de collimation 12 et d'un polariseur 13, d'un dispositif optique d'imagerie 14 et d'un photodétecteur matriciel 15 (capteur d'image).

Les figures 1B et 1C sont des vues schématiques et partielles d'un exemple de la surface fonctionnalisée 5, en perspective (fig.1B) et en coupe (fig.1C). La surface fonctionnalisée 5 est ici une surface d'une couche métallique 3 qui revêt une face du prisme 4. Des ligands adaptés à capter des objets biologiques par adsorption sont disposés dans plusieurs zones distinctes, qui forment alors les sites sensibles (ou sondes) de la surface fonctionnalisée 5. Les ligands peuvent être identiques ou différents d'un site sensible à l'autre.

La figure 1D illustre un exemple d'une courbe SPR, c'est-à-dire une évolution de la réflectivité R en fonction d'un angle d'incidence $\theta$ du signal d'excitation sur la surface fonctionnalisée, ici dans le cadre d'une interrogation dite en réflectivité. La source optique émet un signal d'excitation éclairant le site sensible suivant un angle d'incidence dit de travail $\theta_R$ permettant d'y générer des plasmons de surface de sorte que la sensibilité du système de détection est optimale. On détermine la réflectivité R, c'est-à-dire le rapport de l'intensité du signal de mesure reçu sur l'intensité du signal d'excitation émis. La valeur de la réflectivité R dépend localement de l'indice optique de la surface fonctionnalisée, qui dépend lui-même des plasmons de surface générés et de la quantité de matière adsorbée, cette quantité de matière variant au cours du temps au gré des interactions d'adsorption/désorption avec les ligands.

La figure 1E illustre un exemple d'évolution temporelle de la réflectivité R (également appelée sensorgramme). Le dispositif optique de mesure est préalablement configuré pour générer une résonance plasmonique de surface au niveau de la surface fonctionnalisée lorsque celle-ci est exposée à un liquide (configuration dite en phase liquide, ou en 'mode liquide'). On définit l'angle de travail $\theta_R$ dans une plage angulaire où la sensibilité du système de détection est optimale. Lors d'une première étape, on injecte un liquide de référence, par exemple une solution tampon ne contenant pas les objets biologiques. La réflectivité R présente alors une valeur initiale $R_i$. Lors d'une deuxième étape, on injecte un échantillon liquide formé de la solution tampon contenant maintenant les objets biologiques. Ces derniers se lient alors par adsorption avec les ligands, provoquant alors une variation de l'indice optique à la surface du site sensible qui se traduit par une augmentation de la réflectivité jusqu'à une valeur stationnaire $R_f$. Les objets biologiques adsorbés peuvent alors être caractérisés par la valeur de la variation de réflectivité induite ∆R.

Cependant, il apparaît que la détection d'objets biologiques est particulièrement difficile, et nécessite d'utiliser un système de détection présentant des performances élevées, notamment en termes de sensibilité et de résolution spatiale. En effet, les objets biologiques peuvent présenter un indice de réfraction proche de celui de la solution tampon qui les contient, ce qui implique que le système de détection doit présenter une sensibilité élevée. De plus, ils peuvent présenter une petite taille, par exemple de l'ordre du micron, ce qui rend difficile la détection de chacun des objets biologiques adsorbés sur la surface fonctionnalisée, et demande que le système de détection présente en outre une résolution spatiale importante.

L'article de Laplatine et al. intitulé Spatial resolution in prism-based surface plasmon resonance microscopy, Opt. Express 22(19) 22771-22785 (2014) indique que la résolution spatiale dépend de la longueur de propagation des ondes plasmoniques, mais également d'aberrations optiques liées à la transmission des signaux optiques dans le prisme. Il propose un système de détection optimisé en résolution spatiale par l'utilisation d'un prisme optimisé, d'un système d'imagerie comportant un objectif de grossissement, ainsi qu'une reconstruction du plan image par balayage et traitement d'image. La résolution spatiale obtenue est alors égale à $1.7\mu m$ et à $2.8\mu m$ suivant les axes respectivement perpendiculaire et parallèle au sens de propagation des ondes plasmoniques le tout sur un large champ d'observation.

Par ailleurs, l'article de Boulade et al. intitulé Early detection of bacteria using SPR imaging and event counting: experiments with Listeria monocytogenes and Listeria innocua, RSC Adv., 2019, 9, 15554 décrit l'utilisation d'un système de détection SPR présentant une résolution spatiale optimisée de l'ordre de $6\mu m$ suivant un axe parallèle au sens de propagation des ondes plasmoniques. Une détection de bactéries est effectuée, permettant d'identifier chacune des bactéries adsorbées, lesquelles présentent une taille de l'ordre de grandeur de la résolution spatiale. Deux autres exemples de procédés de détection d'objets biologiques au moyen d'un système de détection par imagerie par résonance plasmonique de surface sont décrits dans les documents de brevet FR 3 103 895 A1 et FR 2 959 568 A1.

Il en résulte que la détection d'objets biologiques de petite taille par imagerie SPR est bien problématique du fait de la résolution spatiale du système de détection. Il existe donc un besoin d'être en mesure de détecter chacun des objets biologiques adsorbés sur les sites sensibles, pour notamment compter ces évènements biologiques d'adsorption, sans avoir recours à un système de détection rendu complexe pour en optimiser la résolution spatiale tout en conservant un large champ d'observation (1 à 100mm$^2$ par exemple). Il existe également un besoin d'être en mesure de détecter des objets biologiques ayant une petite taille au regard de la résolution spatiale.

## EXPOSÉ DE L'INVENTION

L'invention a pour objectif de remédier au moins en partie aux inconvénients de l'art antérieur, et plus particulièrement de proposer un procédé de détection d'objets biologiques au moyen d'un système de détection par imagerie SPR classique dans le sens où il n'est pas nécessaire d'avoir recours à un système de détection complexifié pour en optimiser la résolution spatiale. Le procédé de détection permet également d'identifier des objets biologiques qui présentent une petite taille au regard de la résolution spatiale.

Pour cela, l'objet de l'invention est un procédé de détection , au moyen d'un système de détection par imagerie par résonance plasmonique de surface comportant : une surface fonctionnalisée ayant au moins un site sensible formé de ligands adaptés à se lier à des objets biologiques ; et un dispositif optique de mesure configuré pour générer une résonance plasmonique de surface au niveau de la surface fonctionnalisée lorsque celle-ci est exposée à un gaz, et adapté à acquérir une image du site sensible. Le procédé comporte :

- une étape d'assimilation, comportant une exposition de la surface fonctionnalisée à un échantillon d'intérêt formé d'un liquide porteur aqueux contenant les objets biologiques, les objets biologiques se liant alors aux ligands ;
- une étape d'acquisition d'une image du site sensible par le dispositif optique de mesure ;
- une étape de détection des objets biologiques à partir de l'image acquise.

Selon l'invention, le procédé comprend une étape, effectuée entre l'étape d'assimilation et l'étape d'acquisition, d'évacuation du liquide au contact de la surface fonctionnalisée, et d'exposition de la surface fonctionnalisée à un gaz ne contenant pas les objets biologiques, les objets biologiques restant liés aux ligands, l'étape d'acquisition étant alors effectuée alors que les objets biologiques sont liés aux ligands et que le gaz est au contact de la surface fonctionnalisée.

Certains aspects préférés mais non limitatifs de ce procédé de détection sont les suivants.

Les objets biologiques peuvent présenter une taille inférieure, de préférence au plus 200 fois inférieure, à une résolution spatiale prédéfinie du système de détection.

Les objets biologiques peuvent présenter une taille comprise entre 50nm et $50\mu m$.

Le système de détection peut présenter une résolution spatiale au moins égale à $5\mu m$.

Le procédé de détection peut comporter une étape de rinçage de la surface fonctionnalisée par au moins un liquide, effectuée entre l'étape d'assimilation et l'étape d'évacuation.

Lors de l'étape d'injection fluidique (étape d'assimilation), le liquide porteur de l'échantillon d'intérêt peut être en phase continue ou en phase dispersée.

Lors de l'étape d'évacuation, le gaz peut présenter une humidité relative d'au moins 50%.

Les objets biologiques peuvent être choisis parmi les particules virales, les bactériophages, les bactéries et leurs spores, les archées, les champignons microscopiques et leur spore, les protozoaires unicellulaires, les cellules sanguines ou non circulantes, les vésicules circulantes, les exosomes, les pollens, les objets biologiques comportant une particule synthétique à laquelle est fixée au moins un ligand ou une protéine biologique.

**BRÈVE DESCRIPTION DES DESSINS**

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

la figure 1A, déjà décrite, est une vue en coupe, schématique et partielle, d'un système de détection par imagerie SPR selon un exemple de l'art antérieur ;

les figures 1B et 1C, déjà décrites, sont des vues schématiques et partielles, en perspective et en coupe, de la surface fonctionnalisée du système de détection de la fig.1A ;

la figure 1D, déjà décrite, illustre un exemple de courbes SPR, c'est-à-dire d'évolutions de la réflectivité R en fonction de l'angle d'incidence $\theta$ du signal d'excitation ;

la figure 1E, déjà décrite, illustre un exemple de sensorgramme, c'est-à-dire d'une évolution temporelle de la réflectivité R, lors d'une configuration préalable en 'mode liquide' du dispositif optique de mesure du système de détection, et lors d'une étape d'assimilation d'un procédé de détection des objets biologiques ;

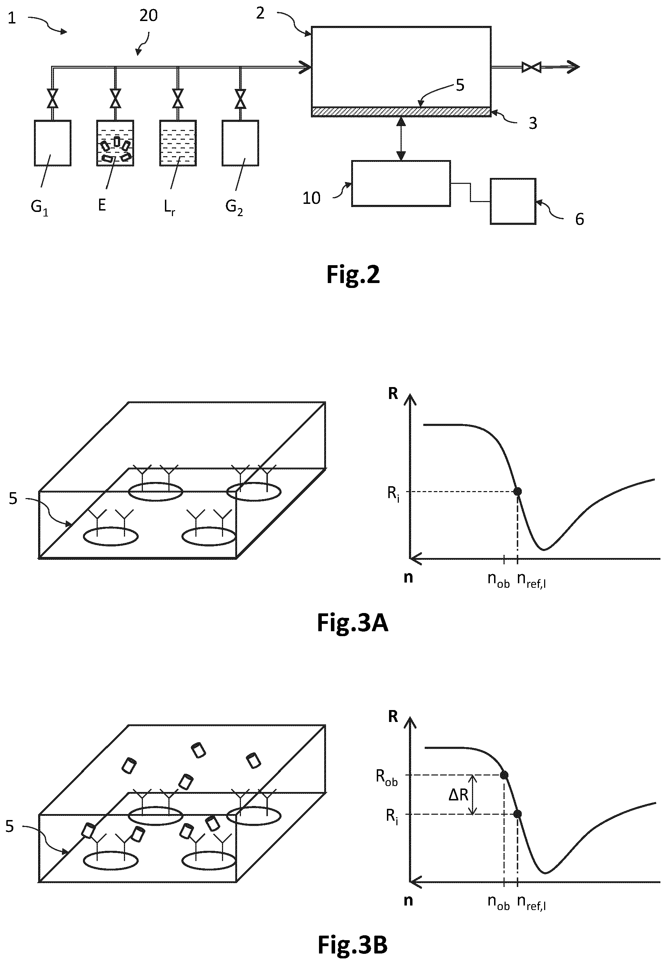

la figure 2 est une vue schématique et partielle d'un système de détection utilisé dans un procédé de détection selon un mode de réalisation ;

les figures 3A et 3B illustrent chacune une surface fonctionnalisée et une courbe SPR (ici évolution de la réflectivité R en fonction de l'indice de réfraction n local), lors d'une configuration préalable en 'mode liquide' du dispositif optique de mesure (fig.3A), et lors d'une étape d'assimilation (fig.3B) d'un procédé de détection où il est nécessaire d'utiliser un système de détection optimisé en résolution spatiale ;

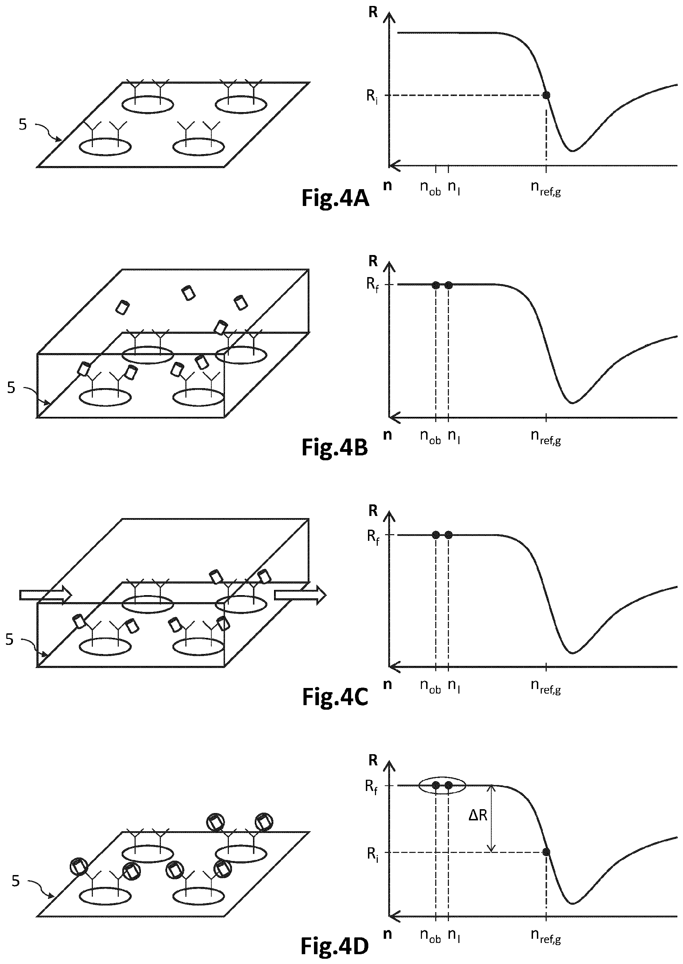

les figures 4A à 4D illustrent chacune une surface fonctionnalisée et une courbe SPR (ici évolution de la réflectivité R en fonction de l'indice de réfraction n local), associées à un système de détection aux performances standard en termes de résolution spatiale et ici configuré en 'mode gaz' (fig.4A), et pour différentes étapes d'un procédé de détection selon un premier mode de réalisation, à savoir : une étape d'assimilation (fig.4B), une étape de rinçage (fig.4C), et une étape d'évacuation et d'acquisition d'une image de détection (fig.4D) ;

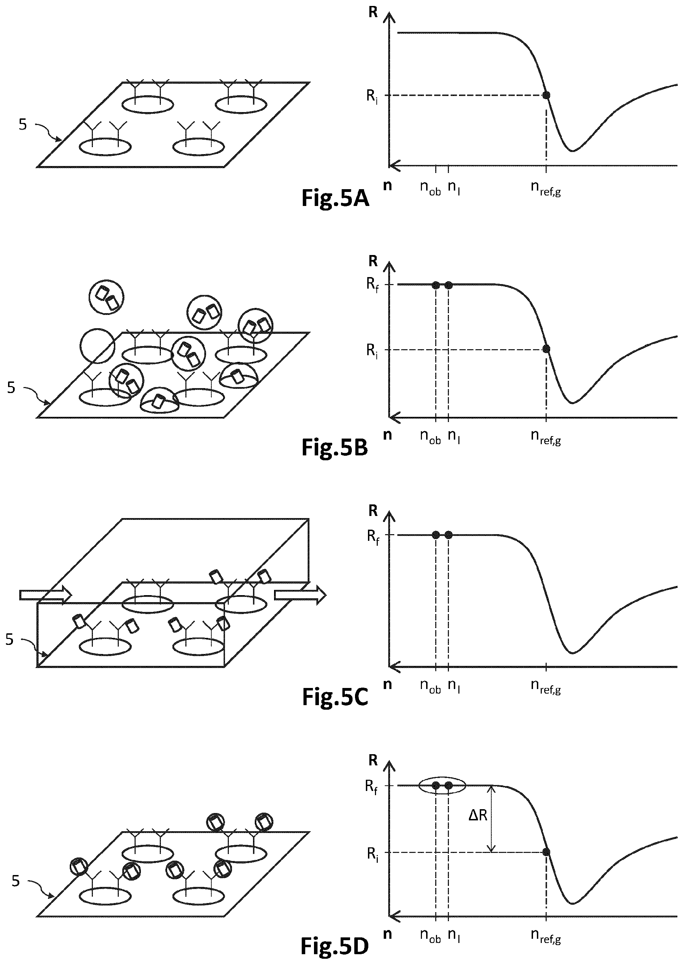

les figures 5A à 5D illustrent chacune une surface fonctionnalisée et une courbe SPR (ici évolution de la réflectivité R en fonction de l'indice de réfraction n local), associées à un système de détection aux performances standard en termes de résolution spatiale et ici configuré en 'mode gaz' (fig.5A), et pour différentes étapes d'un procédé de détection selon un deuxième mode de réalisation à savoir : une étape d'assimilation (fig.5B), une étape de rinçage (fig.5C), et une étape d'évacuation et d'acquisition d'une image de détection (fig.5D) ;

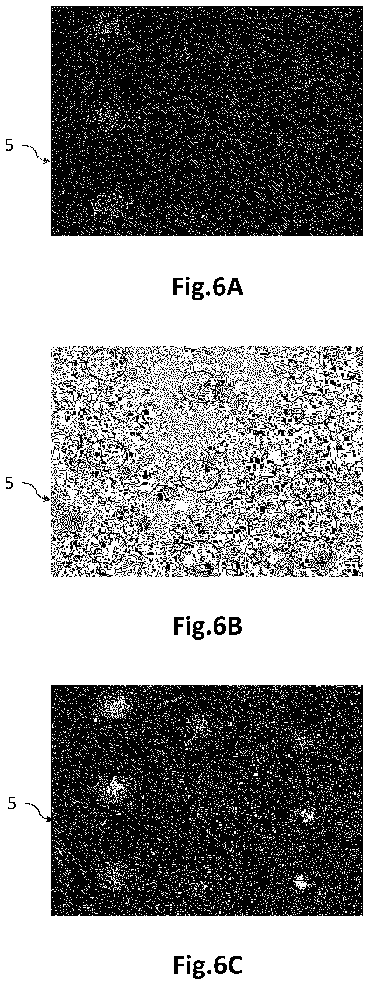

les figures 6A à 6C illustrent des images acquises lors de différentes étapes du procédé de détection selon le premier mode de réalisation, à savoir : avant l'étape d'assimilation (fig.6A), lors l'étape de rinçage faisant suite à l'étape d'assimilation (fig.6B), et lors de l'étape d'évacuation et d'acquisition de l'image de détection (fig.6C), où les objets biologiques sont des particules virales de SARS-CoV-2 ;

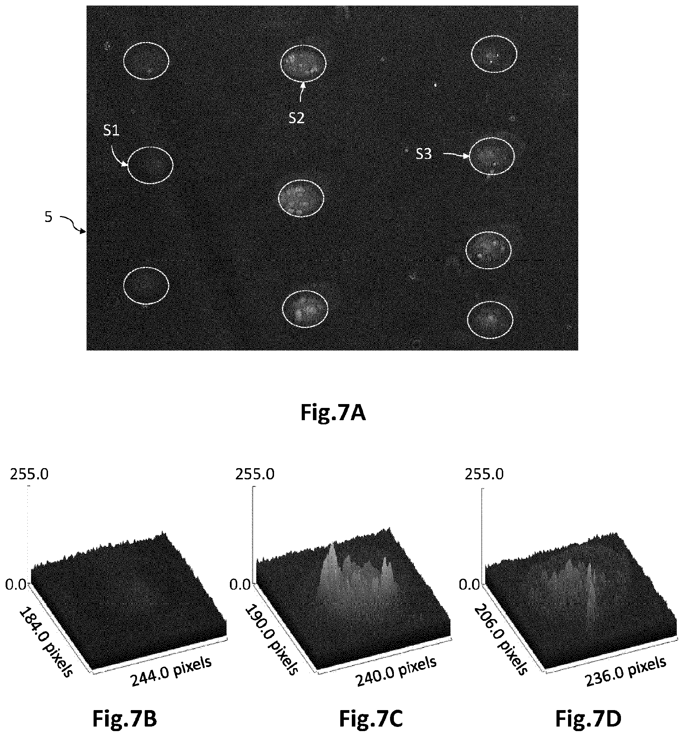

la figure 7A illustre une image de détection acquise dans le cadre du procédé de détection selon le premier mode de réalisation où les objets biologiques sont des particules virales de SARS-CoV-2 ; et les figures 7B à 7D illustrent des sites sensibles dont les ligands sont de différents types ;

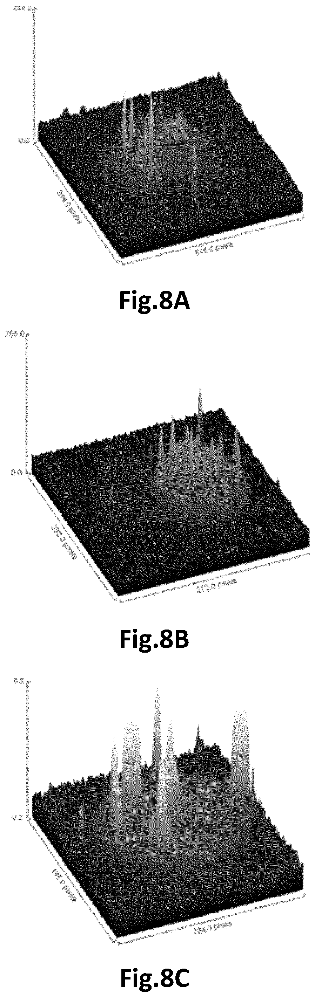

les figures 8A à 8C illustrent chacune un site sensible d'une image de détection acquise dans le cadre du procédé de détection selon le premier mode de réalisation, pour différents types d'objets biologiques, à savoir des billes de polystyrène modifiées (fig.8A), des particules virales de SARS-CoV-2 (fig.8B), et des bactéries (fig.8C).

## EXPOSÉ DÉTAILLÉ DE MODES DE RÉALISATION PARTICULIERS

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux. Sauf indication contraire, les termes « sensiblement », « environ », « de l'ordre de » signifient à 10% près, et de préférence à 5% près. Par ailleurs, les termes « compris entre ... et ... » et équivalents signifient que les bornes sont incluses, sauf mention contraire.

La figure 2, en lien avec les fig.1A et 1B déjà décrites succinctement, illustre un système de détection 1 utilisé dans le cadre d'un procédé de détection selon un mode de réalisation. Le système de détection 1 comporte donc une surface fonctionnalisée 5 située ici dans une chambre de mesure 2, un dispositif optique de mesure 10, éventuellement une unité de traitement 6, et éventuellement un dispositif de gestion fluidique 20.

Comme détaillé plus loin, le procédé de détection selon l'invention permet d'utiliser un système de détection 1 aux performances standard, notamment en termes de résolution spatiale, et permet de détecter chacun des objets biologiques adsorbés à la surface fonctionnalisée 5, alors même qu'ils peuvent présenter une taille inférieure à la résolution spatiale. Il est alors possible de compter les objets biologiques adsorbés, et éventuellement de les caractériser en termes d'affinité vis-à-vis des différents ligands présents.

La résolution spatiale est définie comme étant la distance minimale entre deux points de la surface fonctionnalisée qui peuvent être distingués par le système de détection 1. Elle est notamment limitée par la longueur de propagation $L_x$ de l'onde plasmonique et par les éventuelles aberrations optiques associées à la transmission des signaux optiques au travers du prisme du dispositif optique de mesure 10. Les systèmes de détection standard présentent généralement une résolution spatiale de l'ordre de 5 à 10$\mu$m environ. Ces systèmes de détection par imagerie SPR peuvent être optimisés en résolution spatiale, comme décrit dans l'article de Laplatine et al. 2014 mentionné précédemment et ainsi présenter une résolution spatiale de l'ordre de quelques microns. Le procédé de détection mis en œuvre selon l'invention permet l'utilisation de systèmes de détection standard alors même qu'il s'agit de détecter des objets biologiques dont la taille peut être nettement inférieure à la résolution spatiale.

Les objets biologiques destinés à être détectés sont ici des objets biologiques naturels tels que des particules virales telles que le SARS-CoV-2, complètes ou incomplètes, des bactériophages, des bactéries, des spores de bactéries, des archées, des champignons microscopiques (levures et moisissures) et leur spore, des protozoaires unicellulaires, des cellules sanguines ou non circulantes, des vésicules circulantes, des exosomes, des pollens, des objets biologiques synthétiques comme des nano ou microparticules décorées de ligands ou protéines biologiques (particules biotinylées, recouvertes d'anticorps par ex.). Ils peuvent être des organismes procaryotes ou eucaryotes, uni ou pluricellulaires, d'origine végétale, animale ou humaine. Le microorganisme peut être vivant, c'est-à-dire qu'il est capable de se multiplier.

Les objets biologiques peuvent présenter une taille de l'ordre de quelques dizaines de nanomètres à quelques dizaines de microns, par exemple comprise entre 50nm environ et 50$\mu$m environ, et par exemple comprise entre 100nm et 10$\mu$m. La taille d'un objet biologique est définie comme sa dimension maximale, par exemple le diamètre lorsqu'il présente une forme circulaire, ou sa grande dimension lorsqu'il est de forme allongée. Ainsi, la taille peut être de l'ordre de la centaine de nanomètres dans le cas des virus, et peut être de l'ordre du micromètre dans le cas des bactéries.

La taille des objets biologiques peut être inférieure, sensiblement égale, voire supérieure à la résolution spatiale du système de détection 1. De préférence, les objets biologiques présentent une taille inférieure à la résolution spatiale, de préférence au plus 200 fois inférieure ou au plus 100 fois inférieure, voire encore au plus 50 ou 10 fois inférieure. A titre d'exemple, dans le cas d'une résolution spatiale égale à 10$\mu$m environ (suivant l'axe de propagation de l'onde plasmonique), la taille des objets biologiques peut être comprise entre 50nm environ et 10$\mu$m, de préférence comprise entre 100nm, 200nm, 500nm, 1$\mu$m... et 10$\mu$m. Comme détaillé par la suite, malgré le fait que la taille des objets biologiques peut être inférieure à la résolution spatiale, le procédé de détection selon l'invention autorise d'utiliser un système de détection standard en termes de résolution spatiale, c'est-à-dire qui ne présente pas nécessairement une résolution spatiale optimisée.

Les objets biologiques sont contenus dans le liquide porteur aqueux d'un échantillon d'intérêt, et non pas dans un échantillon gazeux sans liquide porteur. Le liquide porteur peut notamment être une solution tampon. Plus largement, l'échantillon d'intérêt peut être un échantillon biologique (provenant d'organismes vivants ou ayant vécu), alimentaire (provenant d'aliments), d'eau (eau usée, eau douce...), ou de liquides de culture de virus ou de microorganismes, entre autres. Le liquide porteur de l'échantillon d'intérêt peut se présenter en phase continue de sorte que l'échantillon d'intérêt est un échantillon liquide, ou peut se présenter en phase dispersée comme des gouttelettes contenant les objets biologiques situées dans une phase dispersante liquide (l'échantillon est alors dit liquide) ou gazeuse (l'échantillon est alors de type brume ou aérosol).

Le système de détection 1 comporte donc une surface fonctionnalisée 5 d'une couche métallique 3 (homogène ou non), formée d'au moins un site sensible (sonde), et ici d'une pluralité de sites sensibles distincts les uns des autres. La surface fonctionnalisée 5 est ici située sur une face supérieure d'un prisme 3. Les sites sensibles comportent des ligands aptes à interagir avec les objets biologiques à détecter. Les sites sensibles peuvent être identiques ou différents les uns des autres, en termes d'affinité des ligands avec des objets biologiques à détecter. La surface fonctionnalisée 5 peut être passivée par des procédés connus de l'homme du métier, notamment via des PEG (polyéthylène glycol) ou des protéines telles que l'albumine de sérum bovin. La surface fonctionnalisée 5 est ici située dans une chambre de mesure 2, mais en variante elle peut ne pas être située dans une chambre fluidique, et être ainsi exposée à un gaz environnant.

Les ligands sont adaptés à capter (se lier) de manière spécifique les objets biologiques à détecter. On peut se référer au document WO2012073202A1 qui indique des exemples de ligands susceptibles d'être utilisés pour se lier aux objets biologiques. Ainsi, il peut s'agir notamment de récepteurs naturels des objets biologiques, de protéines, de bactériophages ou de virus entiers éventuellement inactivés, d'immunoglobines telles que les anticorps et leurs fragments, de composés synthétiques, entre autres. Il peut également s'agir de DARpines de différentes tailles et composés d'acides aminés naturels ou non et avec ou sans ajout de molécules biologiques, tels que des petites molécules, peptides, protéines, polysaccharides, lipides, ou des groupements chimiques ou des particules. Il peut également s'agir de récepteurs modifiés de l'objet biologique par des approches chimériques, telles que la fusion au domaine Fc des immunoglobulines, par mutagenèse, par insertion d'acide aminé non naturel, par addition de groupements chimiques ou de particules. Il peut également s'agir de peptides synthétiques fixant l'objet biologique incluant des acides aminés naturels ou non naturels avec addition ou non de groupements chimiques ou de particules. Il peut également s'agir d'un substrat clivable par le microorganisme ou par un autre stimulus physicochimique, contenant ou non des domaines, molécules ou des particules additionnelles, incluant des séquences peptidiques, des lipides, des chaînes polysaccharidiques ou de peptidoglycanes. Il peut aussi s'agir de cellules vivantes ou fixées.

Par ailleurs, les ligands présentent une affinité suffisamment forte vis-à-vis des objets biologiques pour que ces derniers restent liés aux ligands lors d'une étape d'évacuation du liquide présent au contact de la surface fonctionnalisée, éventuellement précédée d'une étape de rinçage de celle-ci. A ce titre, rappelons ici que l'interaction entre un analyte A (ici un objet biologique) et un ligand L est un phénomène réversible qui peut être décrit par le modèle de Langmuir, et qui est caractérisé par une constante d'association $k_a$ associé à l'association de l'analyte A avec le ligand L pour former un composé LA (ligand-analyte), et par une constante de dissociation $k_d$ associée à la dissociation du composé LA. Cette relation s'exprime ainsi :

[Math 1]

$$A + L \; \overset{k_a}{\underset{k_d}{\rightleftarrows}} \; AL$$

Dans le cadre de l'invention, l'affinité entre les objets biologiques et les ligands est suffisamment forte pour que les objets biologiques restent liés aux ligands lors des étapes de rinçage et d'évacuation du liquide présent sur la surface fonctionnalisée 5. Autrement dit, le rapport $k_a/k_d$ est supérieur, et de préférence très grand, devant l'unité. De plus, un objet biologique peut être lié à plusieurs ligands d'un même site sensible, ce qui augmente son adhésion à la surface fonctionnalisée 5.

Certains sites sensibles peuvent comporter des récepteurs d'une nature semblable aux ligands mais ne présentant pas d'affinité avec les objets biologiques, pour ainsi servir de témoins négatifs (contrôles négatifs). Autrement dit, ces récepteurs non spécifiques des objets biologiques permettent de déterminer un bruit de mesure, voire une dérive des sondes, et ainsi participent à valider les signaux de mesure reçus.

Le dispositif optique de mesure 10 est adapté à éclairer par un signal d'excitation la surface fonctionnalisée 5 de manière à générer des plasmons de surface, et à recevoir un signal de mesure réfléchi par la surface fonctionnalisée 5 et à acquérir une image de celle-ci et plus précisément des sites sensibles. L'acquisition des images peut être effectuée en temps réel, à une fréquence d'acquisition élevée, ou peut être effectuée lorsqu'on estime que le régime d'association des objets biologiques aux ligands a atteint un régime stationnaire.

Ainsi, le dispositif optique de mesure 10 comporte une source optique 11 adaptée à transmettre le signal optique d'excitation en direction des sites sensibles, avec une longueur d'onde, une polarisation et un angle d'incidence prédéfinis, et ainsi à générer des plasmons de surface au niveau de la surface fonctionnalisée 5. La source optique 11 peut comporter une diode électroluminescente de préférence monochromatique ou une diode laser. Il comporte également des éléments optiques de mise en forme du signal optique d'excitation, tels qu'une ou des lentilles de collimation 12, et un polariseur 13.

Le dispositif optique de mesure 10 peut également comporter des éléments optiques (dispositif optique d'imagerie 14) permettant de conjuguer la surface fonctionnalisée à la face de réception d'un photodétecteur matriciel, permettant ainsi de faire l'image de la surface fonctionnalisée 5 sur le plan de réception du photodétecteur matriciel 15.

Le dispositif optique de mesure 10 comporte un capteur d'image 15, c'est-à-dire un photodétecteur matriciel permettant d'acquérir l'image de la surface fonctionnalisée 5, et plus précisément celle des sites sensibles. Ainsi, les faisceaux lumineux du signal de mesure provenant des sites sensibles sont détectés ensemble et en temps réel, sous la forme d'une image acquise par le même capteur d'image 15. Le capteur d'image 15 peut être un capteur CCD ou CMOS. Il comporte une matrice de pixels dont la résolution spatiale est telle que plusieurs pixels acquièrent le signal de mesure provenant d'un même site sensible. A titre d'exemple, une dimension d'un site sensible de $300\mu$m environ peut être couverte par 150 pixels environ.

Le système de détection 1 peut comporter une unité de traitement 6, connectée au dispositif optique de mesure 10 pour éventuellement effectuer un traitement des images acquises par le capteur d'image 15 pour faciliter la détection des objets biologiques, par exemple en effectuant un filtrage améliorant la netteté des images acquises, voire pour moyenner plusieurs images élémentaires pour former une image finale à partir de laquelle les objets biologiques sont détectés, comme décrit notamment dans la demande WO2020/141281A1. L'unité de traitement 6 peut également être connectée au dispositif de gestion fluidique, pour mettre en œuvre au moins une partie des étapes du procédé de détection.

Le procédé de détection selon l'invention comporte des étapes d'exposition successives de la surface fonctionnalisée 5 à différents fluides. Par exposer, on entend que le fluide vient au contact de la surface fonctionnalisée 5. Ces étapes d'exposition peuvent être effectuées de manière contrôlée par un dispositif fluidique dédié tel que celui illustré sur la fig.2. Les fluides peuvent ainsi être amenés de manière active, par exemple au moyen d'une pompe, d'une seringue etc... Cependant, en variante, ces étapes d'exposition peuvent être effectuées sans avoir recours à un dispositif fluidique dédié. Ainsi, les gaz peuvent être amenés au contact de la surface fonctionnalisée 5 de manière naturelle, par exemple par simple diffusion ou convection naturelle. De plus, le ou les liquides peuvent être amenés au contact de la surface fonctionnalisée 5 en introduisant celle-ci (et ici le prisme) dans le liquide en question. De même, l'étape d'évacuation du liquide présent sur la surface fonctionnalisée 5 peut être effectuée par un dispositif fluidique dédié, comme décrit ci-après, ou peut être effectuée par l'utilisateur, par exemple par écoulement gravitaire, aspiration à l'aide d'une seringue, injection d'un flux gazeux, par pompage capillaire, etc...

Dans cet exemple, le système de détection 1 comporte un dispositif de gestion fluidique 20 adapté à exposer la surface fonctionnalisée 5 à différents fluides de manière successive. Dans cet exemple, la configuration préalable du dispositif optique de mesure 10 en 'mode gaz' est effectuée à l'aide du dispositif fluidique 20. Bien entendu, tout autre dispositif fluidique peut être utilisé. Ainsi, dans cet exemple, le dispositif de gestion fluidique 20 est adapté à exposer la surface fonctionnalisée 5 à un premier gaz $G_1$, dit gaz de référence, à partir d'une source telle qu'ici un réservoir ou à partir de l'environnement du système de détection 1 (auquel cas le gaz de référence $G_1$ peut être l'air ambiant). Ce gaz de référence $G_1$ est utilisé pour déterminer l'angle de travail d'incidence du signal d'excitation générant des plasmons de surface de sorte que le système de détection 1 présente une sensibilité optimale. Notons que la sensibilité du système de détection 1 est ici la variation relative du signal mesuré, ici de la réflectivité R, en réponse à un évènement d'adsorption ou de désorption d'un objet biologique. Le gaz de référence $G_1$ peut être de l'air sec, de l'air humide (par ex. l'air ambiant), ou tout autre gaz tel que de l'argon ou de l'azote.

Le dispositif de gestion fluidique 20 est également adapté à exposer la surface fonctionnalisée 5, lors d'une étape suivante dite d'assimilation, à un échantillon d'intérêt E contenant un liquide porteur aqueux dans lequel sont présents les objets biologiques, à partir d'une source comme ici un réservoir. Comme indiqué précédemment, le liquide porteur peut être en phase continue ou en phase dispersée.

Le dispositif de gestion fluidique 20 peut être adapté à évacuer l'échantillon d'intérêt hors de la chambre de mesure 2 et donc de la surface fonctionnalisée 5. Cela peut être effectué lors d'une étape de rinçage effectuée après l'étape d'assimilation, au cours de laquelle la surface fonctionnalisée 5 est exposée à au moins un liquide de rinçage $L_r$ (solution tampon, détergent, eau ultrapure...) de sorte qu'il s'écoule sur la surface fonctionnalisée 5. Le ou les liquides de rinçage $L_r$ permettent d'évacuer toutes particules, sels ou autres, présents sur la surface fonctionnalisée 5 et non liés à celle-ci de manière spécifique, et susceptibles de générer un signal parasite. Le ou les liquides de rinçage $L_r$ sont de préférence des liquides aqueux. Rappelons ici que les objets biologiques reconnus de façon spécifique restent liés aux ligands lors de cette étape de rinçage, et ne sont donc pas évacués hors de la chambre de mesure 2. En variante, comme indiqué précédemment, cette étape d'évacuation peut être effectuée par l'utilisateur et non pas par le dispositif fluidique 20.

Le dispositif de gestion fluidique 20 est adapté à évacuer tout liquide restant au contact de la surface fonctionnalisée 5, que ce soit un liquide de l'échantillon d'intérêt ou un liquide de rinçage, et à exposer la surface fonctionnalisée 5 à un deuxième gaz $G_2$. De préférence, comme c'est le cas sur l'exemple de la fig.2, le deuxième gaz $G_1$ est identique au gaz de référence $G_1$, et peut ainsi être de l'air humide ou sec, voire de l'argon ou de l'azote. Il peut être introduit dans la chambre de mesure 2 de manière active (par ex. injection du gaz) ou passive (par ex. diffusion ou convection naturelle). Les objets biologiques sont alors dans l'environnement de ce deuxième gaz, alors au contact de la surface fonctionnalisée. En variante, comme indiqué précédemment, cette étape d'évacuation peut être effectuée par l'utilisateur et non pas par le dispositif fluidique 20.

Avant de présenter le procédé de détection selon des modes de réalisation de l'invention, nous détaillons maintenant, en référence aux figures 3A et 3B, un procédé de détection d'objets biologiques qui illustre la nécessité, dans ce cas, de disposer d'un système de détection aux performances améliorées, notamment en termes de résolution spatiale et de sensibilité.

Chacune des figures 3A et 3B illustrent, à gauche, une surface fonctionnalisée 5, et à droite une courbe de l'évolution de la réflectivité R associée au signal de mesure reçu en fonction de l'indice de réfraction local au niveau de la surface fonctionnalisée. Cette courbe est donc équivalente à la courbe SPR classique illustrée sur la fig.1E.

La fig.3A illustre une configuration préalable du dispositif optique de mesure 10, de sorte qu'il génère des plasmons de surface au niveau de la surface fonctionnalisée 5 lorsque celle-ci est exposée à un liquide (configuration en 'mode liquide'). Ainsi, on expose la surface fonctionnalisée 5 à un liquide de référence (par ex. une solution tampon) ne contenant pas les objets biologiques, et la valeur de l'angle de travail $\theta_R$ est ensuite choisie de sorte que le dispositif optique de mesure 10 présente une sensibilité optimale. On se situe dans le cadre d'une détection par interrogation en réflectivité, dans le sens où la longueur d'onde du signal d'excitation et l'angle de travail $\theta_R$ restent inchangés.

Comme l'indique la courbe SPR, la réflectivité R mesurée au niveau des sites sensibles présente une valeur $R_i$ qui est principalement associée à l'indice de réfraction $n_{ref,l}$ du liquide de référence (et bien entendu à celui des ligands). La valeur $R_i$ est sensiblement homogène au niveau de chaque site sensible, qui peut présenter ici une taille circulaire d'un diamètre égal à $300\mu m$ environ. Par ailleurs, le liquide de référence peut être une solution tampon d'indice de réfraction $n_{ref,l}$ égal à 1.33.

La fig.3B illustre une étape d'assimilation du procédé de détection d'objets biologiques, dans laquelle on expose la surface fonctionnalisée 5 à l'échantillon d'intérêt, lequel est ici formé du liquide porteur et des objets biologiques. Le liquide porteur peut être similaire ou identique au liquide de référence, et est ici la même solution tampon. Les objets biologiques présentent un indice de réfraction $n_{ob}$ qui peut être proche de celui du liquide porteur, par exemple égal à 1.4 alors que celui du liquide porteur est de 1.33, soit un écart de 0.07. De plus, les objets biologiques peuvent présenter une petite taille, comme par exemple des virus d'une taille de 100nm environ, ou des bactéries d'une taille de $1\mu m$ environ. Au cours de cette étape, les objets biologiques viennent se lier aux ligands.

Comme l'indique la courbe SPR, la réflectivité R mesurée au niveau de chaque site sensible présente deux valeurs, une valeur localisée $R_{ob}$ associée aux objets biologiques adsorbés, et une valeur continue $R_i$ associée au liquide porteur autour des objets biologiques. Autrement dit, sur une image acquise par le dispositif optique de mesure 10, on peut observer au niveau de chaque site sensible un fond spatialement homogène de valeur $R_i$ et des tâches plus claires de petites dimensions de valeur $R_{ob}$.

Il apparaît cependant que le système de détection 1 doit présenter une sensibilité particulièrement importante pour être en mesure de détecter les objets biologiques du fait du très faible écart, ici égal à 0.07, entre les indices de réfraction $n_{ob}$ et $n_{ref,l}$. Une sensibilité insuffisante se traduirait par une variation de réflectivité $\Delta R$ trop faible, qui ne permettrait pas de distinguer un signal de mesure associé à l'adsorption d'un objet biologique vis-à-vis du bruit de mesure (variations de réflectivité non associées à l'adsorption d'un objet biologique). Autrement dit, le contraste C, défini comme le rapport $(R_{ob}-R_i)/(R_{ob}+R_i)$ entre la réflectivité $R_{ob}$ mesurée au niveau de l'objet biologique et la réflectivité $R_i$ mesurée dans le site sensible hors des objets biologiques, peut être insuffisant pour effectuer une détection effective des objets biologiques.

De plus, les tâches plus claires de réflectivité $R_{ob}$ présentent des dimensions proches de celles de l'objet biologique, ce qui nécessite de disposer d'un système de détection présentant une résolution spatiale très élevée si l'on souhaite être en mesure de détecter chacun des objets biologiques adsorbés sur un site sensible pour les compter. En effet, dans le cas ici d'une bactérie d'une taille de l'ordre du micromètre, il est nécessaire alors d'utiliser un système de détection à haute résolution spatiale comme celui décrit dans l'article de Laplatine et al. 2014 qui présente une résolution spatiale de l'ordre de $2\mu m$. Quoi qu'il en soit, l'utilisation d'un tel système de détection ne permet pas de distinguer chacun des objets biologiques lorsqu'ils présentent une taille bien inférieure à la résolution spatiale, comme c'est le cas par exemple des virus d'une taille de l'ordre de 100nm environ.

Aussi, contrairement aux procédés de l'art antérieur, le procédé de détection d'objets biologiques selon l'invention permet de détecter chacun des objets biologiques adsorbés sur un site sensible avec un système de détection 1 aux performances standard en termes de sensibilité et de résolution spatiale, alors même que les objets biologiques présentent un faible écart d'indice de réfraction avec le liquide porteur et qu'ils présentent une petite taille, de préférence au plus inférieure à 200 fois la résolution spatiale du système de détection 1. A titre d'exemple, le procédé de détection permet de détecter et de distinguer des virus adsorbés sur un même site sensible, lesquels présentent une taille de l'ordre de 100nm, alors même que la résolution spatiale du système de détection est de l'ordre de $10\mu m$.

Les figures 4A à 4D illustrent des étapes d'un procédé de détection selon un premier mode de réalisation, qui utilise un système de détection illustré sur les fig.1A et 1B et sur la fig.2. Dans cet exemple, l'échantillon d'intérêt contenant les objets biologiques est un échantillon liquide.

La fig.4A illustre une configuration préalable du dispositif optique de mesure 10, de sorte qu'il génère des plasmons de surface au niveau de la surface fonctionnalisée 5 lorsqu'elle est exposée à un gaz (configuration en 'mode gaz'). Cette configuration est dite préalable dans le sens où elle est effectuée avant le procédé de détection. Pour cela, la surface fonctionnalisée 5 est exposée à un gaz de référence $G_1$ introduit dans la chambre de mesure 2. Le gaz de référence peut être introduit par le dispositif de gestion fluidique 20, ou par tout autre dispositif fluidique. Le gaz de référence $G_1$ vient donc au contact de la surface fonctionnalisée 5. Le gaz de référence $G_1$ ne contient pas les objets biologiques, et de préférence ne contient aucun élément susceptible de se lier aux ligands ni de générer un signal de mesure spécifique. Il présente un indice de réfraction $n_{ref,g}$ égal ici à 1. L'angle de travail $\theta_R$ est défini de manière à générer des plasmons de surface de sorte que le système de détection 1 présente une sensibilité optimale.

Comme le montre la courbe SPR, la réflectivité mesurée au niveau des sites sensibles présente une valeur $R_i$ sensiblement homogène spatialement. On note donc ici que le dispositif optique de mesure 10 du système de détection 1 utilisé dans le procédé de détection selon l'invention est configuré en 'mode gaz', contrairement au système de détection utilisé dans le procédé de détection des fig.3A et 3B où le dispositif optique de mesure est configuré en 'mode liquide'.

La fig.4B illustre une étape d'assimilation du procédé de détection. Pour cela, le dispositif de gestion fluidique 20 expose la surface fonctionnalisée 5 à l'échantillon d'intérêt introduit dans la chambre de mesure 2. L'échantillon d'intérêt comporte un liquide porteur aqueux dans lequel sont situés les objets biologiques. Le liquide porteur est ici en phase continue, de sorte que l'échantillon d'intérêt est un échantillon liquide et non pas une brume ou un aérosol. Le liquide porteur présente un indice de réfraction $n_l$ égal par exemple à 1.33 et peut être par exemple une solution tampon. Les objets biologiques présentent un indice de réfraction $n_{ob}$ ici égal à 1.4, et peuvent être par exemple un virus tel que le SARS-CoV-2 d'une taille de 100nm environ. Rappelons ici que la résolution spatiale du système de mesure est, dans cet exemple, de l'ordre de $10\mu m$ suivant le sens de propagation des ondes plasmons générées. Au cours de cette étape, le liquide porteur vient au contact de la surface fonctionnalisée 5, et les objets biologiques se lient aux ligands.

Comme l'indique la courbe SPR, la réflectivité R mesurée au niveau de chaque site sensible présente une même valeur $R_f$, sensiblement homogène sur toute l'étendue spatiale du site sensible, associée à la fois au liquide porteur d'indice de réfraction $n_l$ et aux objets biologiques d'indice de réfraction $n_{ob}$. Cette valeur $R_f$ peut être une valeur maximale de la courbe SPR dans la mesure où l'écart $\Delta n$ entre l'indice de réfraction $n_l$ et l'indice de réfraction $n_{ref,g}$ est important, ici de l'ordre de 0.33. Bien entendu, les objets biologiques ne sont pas détectables lors de cette étape.

La fig.4C illustre une étape suivante de rinçage, facultative mais avantageuse. Le rinçage consiste à faire s'écouler sur la surface fonctionnalisée 5 un liquide de rinçage, ou plusieurs liquides de manière successive, permettant ainsi d'évacuer les particules, objets, molécules etc... non liés de manière spécifique aux ligands et susceptibles d'induire un bruit de mesure ou des signaux parasites (variation de la réflectivité non liée aux objets biologiques à détecter). On améliore ainsi la qualité de la détection et notamment le rapport signal sur bruit SNR en réduisant les sources de signal parasite. A titre d'exemple, lors de l'étape de rinçage, on peut injecter une solution tampon avec un détergent, puis une solution tampon identique au liquide porteur (mais évidemment sans les objets biologiques), et enfin une eau ultrapure pour enlever les éventuels sels présents dans la solution tampon. Rappelons que les objets biologiques restent adsorbés de manière spécifique pendant cette étape de rinçage, du fait de la forte affinité avec les ligands et la faible dissociation.

La fig.4D illustre une étape suivante d'évacuation du liquide au contact de la surface fonctionnalisée 5. Pour cela, le liquide présent est évacué hors de la chambre de mesure 2, par exemple par voie gravitaire, aspiration, capillarité..., et on expose la surface fonctionnalisée 5 à un deuxième gaz $G_2$ qui ne contient évidemment pas d'objets biologiques. Ce deuxième gaz $G_2$ peut être similaire ou identique au gaz de référence utilisé précédemment, notamment en termes de composition chimique, et présente un indice de réfraction proche ou identique à $n_{ref,g}$. Il ne contient de préférence pas de particules ayant un impact sur le signal de mesure. Ici également, les objets biologiques restent adsorbés pendant cette étape d'évacuation, de sorte qu'ils sont situés dans le deuxième gaz qui est au contact de la surface fonctionnalisée (et non pas dans un liquide comme dans le procédé de détection des fig.3A et 3B). De préférence, le deuxième gaz $G_2$ est un gaz humide et présente une humidité relative non nulle et de préférence d'au moins 50%.

A la suite de l'étape d'évacuation, une étape d'acquisition d'une image de détection de la surface fonctionnalisée 5 est effectuée par le dispositif optique de mesure 10. L'image acquise comporte donc les images de chacun des sites sensibles, et les objets biologiques peuvent être détectés.

Comme l'indique la courbe SPR, la réflectivité R mesurée au niveau de chaque site sensible présente deux valeurs, une valeur localisée $R_f$ associée aux objets biologiques adsorbés, et une valeur continue $R_i$ associée au gaz d'indice de réfraction $n_{ref,g}$. Autrement dit, sur l'image acquise par le dispositif optique de mesure 10, on peut observer au niveau de chaque site sensible un fond spatialement homogène de valeur $R_i$ et des tâches bien plus claires de valeur $R_f$. Les tâches claires présentent donc une intensité (réflectivité R) très élevée dans la mesure où la valeur $R_f$ de la réflectivité R est proche voire égale à la valeur maximale possible (saturation de la réflectivité) de la courbe SPR dans la gamme de variation de l'indice de réfraction local n. Ainsi, la détection des objets biologiques est fortement améliorée, dans la mesure où l'écart de réflectivité $\Delta R$, et donc le contraste C, est augmenté par rapport à la situation de la fig.3B, puisque l'écart $\Delta n$ entre les indices de réfraction est maintenant de l'ordre de 0.33 et non plus de 0.07 (soit une augmentation de près de 400%).

De plus, les inventeurs ont constaté que les tâches claires associées aux objets biologiques présentent une étendue surfacique bien supérieure à la taille effective (taille physique) des objets biologiques, par exemple une étendue de l'ordre de 15 à 20μm dans le cas des virus SARS-CoV-2 dont la taille effective est de l'ordre de 100nm. Il semble que cela est dû à la présence d'une fine pellicule d'eau liée à chaque objet biologique, appelée également couche d'eau biologique ou couche d'hydratation d'une part, et à un phénomène optique de diffusion du signal réfléchi par l'objet biologique et de sa pellicule d'eau d'autre part. Rappelons que les molécules d'eau présentent une polarité électrique et peuvent se lier aux objets biologiques comme les virus et les bactéries. Il s'agirait donc d'eau biologique (*biological water,* en anglais), par opposition à l'eau libre (*bulk water,* en anglais) qui est évacuée hors de la chambre de mesure 2. Une telle pellicule d'eau biologique est notamment décrite dans l'article de Pal et al. intitulé Biological water at the protein surface: Dynamical solvation probed directly with femtosecond resolution, PNAS vol.99, no.4, 1763-1768, 2002. Cette pellicule d'eau biologique peut provenir du liquide porteur aqueux de l'échantillon d'intérêt, en particulier lorsque l'étape de rinçage n'est pas effectuée, et/ou du ou des liquides aqueux de rinçage. Elle peut également provenir au moins en partie de molécules d'eau du deuxième gaz introduit lors de l'étape d'évacuation (ce deuxième gaz $G_2$ présente alors de préférence une humidité relative d'au moins 50%). Il en résulte donc qu'il est alors possible de détecter chacun des objets biologiques présents sur un même site sensible, alors même qu'ils présentent une taille (par ex. de l'ordre de 100nm ou de 1μm) bien inférieure à la résolution spatiale (par ex. de l'ordre de 10μm) du système de détection.

Les figures 5A à 5D illustrent des étapes d'un procédé de détection selon un deuxième mode de réalisation au moyen du système de détection 1 configuré en 'mode gaz'. Dans cet exemple, l'échantillon d'intérêt contenant les objets biologiques est une brume formée d'un gaz (phase dispersante), un liquide porteur aqueux sous forme de gouttelettes (phase dispersée) qui contiennent les objets biologiques. Ce mode de réalisation est similaire à celui décrit en référence aux fig.4A à 4D et seules les étapes différentes sont détaillées.

Comme l'illustre la fig.5A, le dispositif optique de mesure 10 est configuré en 'mode gaz', comme pour le procédé de détection selon le premier mode de réalisation (fig.4A), et non pas en 'mode liquide'. Les étapes de rinçage (fig.5C), d'évacuation et d'acquisition (fig.5D) sont ici identiques ou similaires à celles décrites précédemment. En revanche, lors de l'étape d'assimilation (fig.5B) où on expose la surface fonctionnalisée 5 à l'échantillon d'intérêt, celui-ci est introduit dans la chambre de mesure 2 alors que le liquide porteur est en phase dispersée. Ainsi, des gouttelettes, par exemple d'un volume de l'ordre de la centaine de micromètres au cube voire davantage, et contenant des objets biologiques, viennent se déposer sur la surface fonctionnalisée 5, et les objets biologiques peuvent se lier aux ligands.

Comme l'illustre la courbe SPR, la réflectivité R mesurée au niveau de chaque site sensible peut présenter deux valeurs, une valeur $R_f$ associée aux gouttelettes, et une valeur continue $R_i$ associée au gaz (phase dispersante) de l'échantillon d'intérêt. Autrement dit, sur une image acquise par le dispositif optique de mesure 10, on peut observer un fond spatialement homogène de valeur $R_i$ et des tâches plus claires de grandes dimensions de valeur $R_f$ correspondant aux gouttelettes.

Dans ce mode de réalisation, une étape de rinçage peut faciliter l'évacuation du liquide présent au contact de la surface fonctionnalisée 5, pour ensuite effectuer l'étape d'acquisition de l'image permettant de détecter les objets biologiques. Notons que, dans ce mode de réalisation également, à la suite de l'évacuation de tout liquide présent au contact de la surface fonctionnalisée 5, une pellicule d'eau biologique liée est présente au niveau de chaque objet biologique, ce qui permet de distinguer les objets biologiques les uns des autres alors même que leur taille est inférieure à la résolution spatiale du système de détection.

Les figures 6A à 6C sont des images de la surface fonctionnalisée 5 acquises à différentes étapes du procédé de détection selon le premier mode de réalisation. Dans cet exemple, les objets biologiques sont des virus du SARS-CoV-2 inactivés.

La fig.6A illustre une image acquise alors que le gaz de référence $G_1$ est introduit dans la chambre de mesure (cf. fig.4A). On se situe ici en amont de l'étape d'assimilation du procédé de détection. Le gaz de référence $G_1$ est ici de l'air ambiant. La surface fonctionnalisée 5 comporte ici une matrice de 3 ensembles de 3 sites sensibles (délimités ici par une ligne en pointillés), où les ligands sont identiques dans un même ensemble mais différents d'un ensemble à l'autre. Les sites sensibles des colonnes de gauche et de droite comportent des ligands de contrôle positif, c'est-à-dire adaptés à se lier de manière spécifique aux virus, et sont ici des anticorps anti-S pour la colonne de gauche, et des anticorps anti-S pour la colonne de droite. Les sites sensibles de la colonne centrale comportent des récepteurs de contrôle négatif, c'est-à-dire adaptés à ne pas se lier de manière spécifique aux virus, et sont ici des anticorps anti-KLH (pour *Keyhole Limpet Hemocyanin,* en anglais). On observe que, pour l'angle de travail choisi, la réflectivité R est très faible pour chaque site sensible : on se situe ainsi à proximité du creux de réflectivité de la courbe SPR. Le contraste, défini ici comme le rapport $(R_{in,moy}-R_{out})/(R_{in,moy}+R_{out})$ à partir de la réflectivité $R_{in,moy}$ dans un site sensible et de la réflectivité $R_{out}$ en-dehors des sites sensibles, est très faible.

La fig.6B illustre une image acquise lors de l'étape de rinçage, alors qu'un liquide de rinçage (ici de l'eau ultrapure) est au contact de la surface fonctionnalisée 5. Comme le montre la fig.4C, la réflectivité est alors très élevée sur toute la surface fonctionnalisée 5. Il n'est alors pas possible de distinguer les sites sensibles, ni évidemment les virus.

La fig.6C illustre une image acquise lors de l'étape d'acquisition, après évacuation de tout liquide situé au contact de la surface fonctionnalisée 5. On remarque que les sites sensibles de la colonne de gauche présentent des tâches très claires (sites du haut et du milieu), ainsi que les sites sensibles de la colonne de droite (sites du milieu et du bas). En revanche, les sites sensibles de la colonne centrale (contrôle négatif) ne présentent pas de tâches très claires.

En accord avec la fig.4D, la réflectivité présente bien ici une valeur minimale $R_{min}$ en-dehors des sites sensibles; une valeur $R_i$ supérieure à cette valeur minimale $R_{min}$ au niveau des sites sensibles mais en-dehors des virus ; et une valeur $R_f$ très élevée, et proche d'une valeur maximale, là où les virus sont adsorbés. Ainsi, le contraste entre la réflectivité $R_f$ associée aux virus et la réflectivité $R_i$ associée aux sites sensibles hors virus est très élevé et permet de bien détecter les virus, mais il est encore plus élevé entre la réflectivité $R_f$ et la réflectivité $R_{min}$. De plus, les virus apparaissent ici sous forme de tâches très claires dont l'étendue spatiale est de l'ordre de 15 à $20\mu m$, dans les sites sensibles de $300\mu m$ de diamètre, alors même que les virus SARS-Cov-2 présentent une taille de l'ordre de 100nm environ. Ainsi, chaque virus peut être distingué de ses voisins, et un comptage du nombre de virus adsorbés est alors possible alors même que le système de détection présente des performances standard en termes de résolution spatiale (ici de l'ordre de 5 à $10\mu m$). La correspondance entre ces tâches claires et la présence des particules virales a été confirmée par une observation au microscope électronique à balayage (MEB).

La figure 7A et les figures 7B à 7C sont des images de la surface fonctionnalisée 5, acquises à la suite de l'étape d'évacuation du procédé de détection selon le premier mode de réalisation. Dans cet exemple, les objets biologiques sont également des virus du SARS-CoV-2 inactivés.

La fig.7A illustre la surface fonctionnalisée où les sites sensibles de la colonne de gauche comportent des récepteurs de contrôle négatif, ici des anticorps anti-KLH, ceux de la colonne du milieu comportent des ligands de contrôle positif, ici des anticorps anti-N, et ceux de la colonne de droite comportent des ligands de contrôle positif, ici des anticorps anti-S.

On remarque que les sites sensibles aux anticorps anti-KLH ne présentent pas les tâches très claires caractéristiques de la présence des virus, alors que ceux aux anticorps de contrôle positif en présentent.

La fig.7B illustre une vue du site sensible S1 aux anticorps anti-KLH. La réflectivité varie ici sur une échelle de gris en 8 bits, c'est-à-dire qu'elle varie entre 0 et 255. La réflectivité présente une valeur sensiblement homogène très faible, proche de $R_{min}$.

La fig.7C illustre une vue du site sensible S2 aux anticorps anti-N. La réflectivité présente la valeur minimale $R_{min}$ en -dehors du site sensible, ainsi que des pics dans le site sensible représentatifs de la présence de virus adsorbés. On remarque que le contraste est plus élevé, et que les pics présentent une étendue spatiale bien plus élevée que la taille effective des virus.

La fig.7D illustre une vue du site sensible S3 aux anticorps SV205 anti-S. La réflectivité présente également quelques pics représentatifs de la présence de virus adsorbés, mais en nombre moins important que dans la fig.7C.

Les figures 8A à 8C sont des images de la surface fonctionnalisée, acquises à la suite de l'étape d'évacuation du procédé de détection selon le premier mode de réalisation, pour différents exemples d'objets biologiques. Ces images montrent l'évolution spatiale de la réflectivité en échelle de gris en 8 bits.

La fig.8A illustre une image d'un site sensible dont les ligands sont de la biotine. Les objets biologiques sont ici des billes de polystyrène modifiées à la streptavidine et d'une taille de 200nm environ. On remarque que la réflectivité présente des pics localisés représentatifs de l'adsorption des billes de polystyrène modifiées, dont l'intensité et l'étendue spatiale permettent d'effectuer une détection et un comptage.

La fig.8B illustre une image d'un site sensible dont les ligands sont anti-S. Les objets biologiques sont ici des particules virales de SARS-Cov-2 inactivées d'une taille de 100nm environ. La réflectivité présente également des pics localisés représentatifs de la présence de virus adsorbés, dont l'intensité et l'étendue spatiale permettent d'effectuer une détection et un comptage. La modification du niveau moyen de réflectivité associée au site sensible, hors des pics, peut provenir de la présence de protéines virales solubles reconnues par les mêmes ligands.

La fig.8C illustre une image d'un site sensible dont les ligands sont anti-*E. Coli*. Les objets biologiques sont ici des bactéries *E. Coli* K12 d'une taille de 1 à $2\mu m$ environ. La réflectivité présente ici également des pics localisés représentatifs de la présence de bactéries adsorbées, dont l'intensité et l'étendue spatiale permettent d'effectuer une détection et un comptage. Ici, le niveau moyen de réflectivité, hors des pics, est associé à des fragments moléculaires provenant des bactéries ciblées.

Ainsi, il en résulte que le procédé de détection selon l'invention permet de détecter de manière efficace chacun des objets biologiques adsorbés sur les sites sensibles, avec un système de détection aux performances standard en termes de résolution spatiale et de sensibilité. Cela est notamment dû aux faits que, alors que les objets biologiques sont amenés au contact de la surface fonctionnalisée dans un liquide porteur, le dispositif optique de mesure est préalablement configuré en 'mode gaz', et l'acquisition de l'image de détection est effectuée alors que les objets biologiques sont dans un environnement gazeux.

Des modes de réalisation particuliers viennent d'être décrits. Différentes variantes et modifications apparaîtront à l'homme du métier.

**Revendications**

1. Procédé de détection d'objets biologiques, au moyen d'un système de détection (1) par imagerie par résonance plasmonique de surface comportant : une surface fonctionnalisée (5) ayant au moins un site sensible formé de ligands adaptés à se lier à des objets biologiques ; et un dispositif optique de mesure (10) configuré pour générer une résonance plasmonique de surface au niveau de la surface fonctionnalisée (5) lorsque celle-ci est exposée à un gaz, et adapté à acquérir une image du site sensible ; le procédé comportant :

   ◦ une étape d'assimilation, comportant une exposition de la surface fonctionnalisée (5) à un échantillon d'intérêt formé d'un liquide porteur aqueux contenant les objets biologiques, les objets biologiques se liant alors aux ligands ;
   ◦ une étape d'acquisition d'une image du site sensible par le dispositif optique de mesure (10) ;
   ◦ une étape de détection des objets biologiques à partir de l'image acquise ;
   ◦ le procédé étant **caractérisé en ce qu'**il comprend une étape, effectuée entre l'étape d'assimilation et l'étape d'acquisition, d'évacuation du liquide au contact de la surface fonctionnalisée (5), et d'exposition de la surface fonctionnalisée (5) à un gaz ne contenant pas les objets biologiques, les objets biologiques restant liés aux ligands, l'étape d'acquisition étant alors effectuée alors que les objets biologiques sont liés aux ligands et que le gaz est au contact de la surface fonctionnalisée (5).

2. Procédé de détection selon la revendication 1, dans lequel les objets biologiques présentent une taille inférieure, de préférence au plus 200 fois inférieure, à une résolution spatiale prédéfinie du système de détection (1).

3. Procédé de détection selon la revendication 1 ou 2, dans lequel les objets biologiques présentent une taille comprise entre 50nm et 50$\mu$m.

4. Procédé de détection selon l'une quelconque des revendications 1 à 3, dans lequel le système de détection (1) présente une résolution spatiale au moins égale à 5$\mu$m.

5. Procédé de détection selon l'une quelconque des revendications 1 à 4, comportant une étape de rinçage de la surface fonctionnalisée (5) par au moins un liquide, effectuée entre l'étape d'assimilation et l'étape d'évacuation.

6. Procédé de détection selon l'une quelconque des revendications 1 à 5, dans lequel, lors de l'étape d'assimilation, le liquide porteur de l'échantillon d'intérêt est en phase continue ou en phase dispersée.

7. Procédé de détection selon l'une quelconque des revendications 1 à 6, dans lequel, lors de l'étape d'évacuation, le gaz présente une humidité relative d'au moins 50%.

8. Procédé de détection selon l'une quelconque des revendications 1 à 7, dans lequel les objets biologiques sont choisis parmi les particules virales, les bactériophages, les bactéries et leurs spores, les archées, les champignons microscopiques et leur spore, les protozoaires unicellulaires, les cellules sanguines ou non circulantes, les vésicules circulantes, les exosomes, les pollens, les objets biologiques comportant une particule synthétique à laquelle est fixée au moins un ligand ou une protéine biologique.

**Patentansprüche**

1. Verfahren zur Erkennung biologischer Objekte mittels eines Erkennungssystems (1) durch Oberflächenplasmonenresonanzbildgebung, umfassend: eine funktionalisierte Oberfläche (5) mit mindestens einer empfindlichen Stelle, die aus Liganden gebildet ist, die geeignet sind, sich an biologische Objekte zu binden; und eine optische Messvorrichtung (10), die so eingerichtet ist, dass sie eine Oberflächenplasmonenresonanz auf der Ebene der funktionalisierten Oberfläche (5) erzeugt, wenn diese einem Gas ausgesetzt ist, und geeignet ist, ein Bild der empfindlichen Stelle zu erfassen; wobei das Verfahren Folgendes umfasst:

   ◦ einen Aufnahmeschritt, der das Aussetzen der funktionalisierten Oberfläche (5) gegenüber einer interessierenden Probe umfasst, die aus einer wässrigen Trägerflüssigkeit gebildet ist, die die biologischen Objekte enthält, wobei sich die biologischen Objekte anschließend an die Liganden binden;
   ◦ einen Schritt des Erfassens eines Bildes der empfindlichen Stelle durch die optische Messvorrichtung (10);
   ◦ einen Schritt des Erkennens biologischer Objekte aus dem erfassten Bild;

∘ wobei das Verfahren **dadurch gekennzeichnet ist, dass** es einen zwischen dem Aufnahmeschritt und dem Erfassungsschritt durchgeführten Schritt des Ableitens der Flüssigkeit, die in Kontakt mit der funktionalisierten Oberfläche (5) ist, und des Aussetzens der funktionalisierten Oberfläche (5) gegenüber eines Gases, das die biologischen Objekte nicht enthält, umfasst, wobei die biologischen Objekte an den Liganden gebunden bleiben, wobei der Erfassungsschritt anschließend durchgeführt wird, während die biologischen Objekte an den Liganden gebunden sind und das Gas mit der funktionalisierten Oberfläche (5) in Kontakt ist.

2. Verfahren zur Erkennung nach Anspruch 1, wobei die biologischen Objekte eine kleinere Größe aufweisen, vorzugsweise höchstens das 200-Fache einer vordefinierten räumlichen Auflösung des Erkennungssystems (1).

3. Verfahren zur Erkennung nach Anspruch 1 oder 2, wobei die biologischen Objekte eine Größe zwischen 50 nm und 50 $\mu$m aufweisen.

4. Verfahren zur Erkennung nach einem der Ansprüche 1 bis 3, wobei das Erkennungssystem (1) eine räumliche Auflösung von mindestens 5 $\mu$m aufweist.

5. Verfahren zur Erkennung nach einem der Ansprüche 1 bis 4, das einen Schritt des Spülens der funktionalisierten Oberfläche (5) mit mindestens einer Flüssigkeit umfasst, der zwischen dem Aufnahmeschritt und dem Ableitungsschritt durchgeführt wird.

6. Verfahren zur Erkennung nach einem der Ansprüche 1 bis 5, wobei sich die Trägerflüssigkeit der betreffenden Probe während des Aufnahmeschritts in der kontinuierlichen Phase oder in der dispersen Phase befindet.

7. Verfahren zur Erkennung nach einem der Ansprüche 1 bis 6, wobei das Gas während des Ableitungsschritts eine relative Feuchtigkeit von mindestens 50 % aufweist.

8. Verfahren zur Erkennung nach einem der Ansprüche 1 bis 7, wobei die biologischen Objekte aus Viruspartikeln, Bakteriophagen, Bakterien und ihren Sporen, Archaeen, mikroskopischen Pilzen und ihren Sporen, einzelligen Protozoen, Blutzellen oder nicht zirkulierenden Blutzellen, zirkulierenden Vesikeln, Exosomen, Pollen ausgewählt sind, wobei die biologischen Objekte ein synthetisches Teilchen umfassen, an das mindestens ein biologischer Ligand oder ein biologisches Protein gebunden ist.

**Claims**

1. Method for detecting biological objects by means of a surface plasmon resonance imaging detection system (1) comprising: a functional surface (5) having at least one sensitive site formed of ligands adapted to bind to biological objects; and an optical measurement device (10) configured to generate surface plasmon resonance at the functional surface (5) when the functional surface is exposed to a gas, and adapted to acquire an image of the sensitive site; the method comprising:

∘ an assimilation step, comprising exposing the functional surface (5) to a sample of interest formed of an aqueous carrier liquid containing the biological objects, the biological objects thus binding to the ligands;
∘ a step of acquiring an image of the sensitive site using the optical measuring device (10);
∘ a step of detecting the biological objects from the acquired image;
∘ the method being **characterised in that** it comprises a step, which is carried out between the assimilation step and the acquisition step, of removing the liquid in contact with the functional surface (5), and of exposing the functional surface (5) to a gas not containing the biological objects, the biological objects remaining bound to the ligands, the acquisition step thus being carried out while the biological objects are bound to the ligands and while the gas is in contact with the functional surface (5).

2. Detection method according to claim 1, wherein the biological objects are smaller in size, preferably up to 200 times smaller, than a predefined spatial resolution of the detection system (1).

3. Detection method according to claim 1 or 2, wherein the biological objects have a size between 50 nm and 50 $\mu$m.

4. Detection method according to any one of claims 1 to 3, wherein the detection system (1) has a spatial resolution at least equal to 5 $\mu$m.

**5.** Detection method according to any one of claims 1 to 4, comprising a step of rinsing the functional surface (5) using at least one liquid, which step is carried out between the assimilation step and the removal step.

**6.** Detection method according to any one of claims 1 to 5, wherein, during the assimilation step, the liquid carrying the sample of interest is in continuous phase or dispersed phase.

**7.** Detection method according to any one of claims 1 to 6, wherein, during the removal step, the gas has a relative humidity of at least 50%.

**8.** Detection method according to any one of claims 1 to 7, wherein the biological objects are selected from viral particles, bacteriophages, bacteria and their spores, archaea, microscopic fungi and their spores, single-celled protozoa, blood cells or non-circulating cells, circulating vesicles, exosomes, pollen, biological objects comprising a synthetic particle to which at least one biological protein or ligand is attached.

**Fig.1A**

**Fig.1B**

**Fig.1C**

**Fig.1D**

**Fig.1E**

**Fig.2**

**Fig.3A**

**Fig.3B**

Fig.4A

Fig.4B

Fig.4C

Fig.4D

Fig.5A

Fig.5B

Fig.5C

Fig.5D

**Fig.6A**

**Fig.6B**

**Fig.6C**

**Fig.7A**

**Fig.7B**

**Fig.7C**

**Fig.7D**

**Fig.8A**

**Fig.8B**

**Fig.8C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018158458 A1
- FR 3103895 A1
- FR 2959568 A1
- WO 2012073202 A1
- WO 2020141281 A1

**Littérature non-brevet citée dans la description**

- **LAPLATINE et al.** Spatial resolution in prism-based surface plasmon resonance microscopy. *Opt. Express*, 2014, vol. 22 (19), 22771-22785
- **BOULADE et al.** Early detection of bacteria using SPR imaging and event counting: experiments with Listeria monocytogenes and Listeria innocua. *RSC Adv.*, 2019, vol. 9, 15554
- **PAL et al.** Biological water at the protein surface: Dynamical solvation probed directly with femtosecond resolution. *PNAS*, 2002, vol. 99 (4), 1763-1768